(19) Europäisches Patentamt
European Patent Office
Office européen des brevets

(11) EP 0 520 722 B1

(12) **EUROPEAN PATENT SPECIFICATION**

(45) Date of publication and mention of the grant of the patent:
**27.12.1996 Bulletin 1996/52**

(51) Int Cl.[6]: **C07D 239/94**, A61K 31/505

(21) Application number: **92305703.8**

(22) Date of filing: **22.06.1992**

(54) **Therapeutic preparations containing quinazoline derivatives**

Chinazolinderivate enthaltende therapeutische Zusammensetzungen

Préparations thérapeutiques contenant des dérivés de quinazoline

(84) Designated Contracting States:
**AT BE CH DE DK FR GB GR IT LI LU MC NL PT SE**

(30) Priority: **28.06.1991 GB 9113970**
**20.01.1992 GB 9201133**

(43) Date of publication of application:
**30.12.1992 Bulletin 1992/53**

(73) Proprietor: **ZENECA LIMITED**
**London W1Y 6LN (GB)**

(72) Inventors:
- **Barker, Andrew John**
  **Macclesfield, Cheshire SK10 4TG (GB)**
- **Davies, David Huw**
  **Macclesfield, Cheshire SK10 4TG (GB)**

(74) Representative: **Tait, Brian Steele et al**
**Intellectual Property Department**
**ZENECA Pharmaceuticals**
**Mereside**
**Alderley Park**
**Macclesfield Cheshire SK10 4TG (GB)**

(56) References cited:
**GB-A- 2 033 894**

- **PATENT ABSTRACTS OF JAPAN, vol. 6, no. 244 (C-138)(1122) 2 December 1982**
- **PATENT ABSTRACTS OF JAPAN, vol. 4, no. 73 (C-12)(555) 28 May 1980**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 520 722 B1

## Description

The invention relates to therapeutic preparations and more particularly it relates to pharmaceutical compositions of quinazoline derivatives, or pharmaceutically-acceptable salts thereof, which possess anti-cancer activity. The invention also relates to certain novel quinazoline derivatives and to processes for their manufacture. The invention also relates to the use of either the novel quinazoline derivatives of the invention or certain known quinazoline derivatives in the manufacture of medicaments for use in the production of an anti-cancer effect in a warm-blooded animal such as man.

Many of the current treatment regimes for cancer utilise compounds which inhibit DNA synthesis. Such compounds are toxic to cells generally but their effect on the rapidly dividing tumour cells can be beneficial. Alternatie approaches to anti-cancer agents which act by mechanisms other than the inhibition of DNA synthesis have the potential to display enhanced selectivity of action against cancer cells.

In recent years it has been discovered that a cell may become cancerous by virtue of the transformation of a portion of its DNA into an oncogene i.e. a gene which, on activation, leads to the formation of malignant tumour cells (Bradshaw, Mutagenesis, 1986, 1, 91). Several such oncogenes give rise to the production of peptides which are receptors for growth factors. The growth factor receptor complex subsequently leads to an increase in cell proliferation. It is known, for example, that several oncogenes encode tyrosine kinase enzymes and that certain growth factor receptors are also tyrosine kinase enzymes (Yarden et al., Ann. Rev. Biochem., 1988, 57, 443; Larsen et al. Ann. Reports in Med. Chem. 1989, Chpt. 13).

Receptor tyrosine kinases are important in the transmission of biochemical signals which initiate cell replication. They are large enzymes which span the cell membrane and possess an extracellular binding domain for growth factors such as epidermal growth factor and an intracellular portion which functions as a kinase to phosphorylate tyrosine amino acids in proteins and hence to influence cell proliferation. It is known that such kinases are frequently present in common human cancers such as breast cancer (Sainsbury et al., Brit. J. Cancer, 1988, 58, 458; Guerin et al., Oncogene Res., 1988, 3, 21), gastrointestinal cancer such as colon, rectal or stomach cancer (Bolen et al., Oncogene Res., 1987, 1, 149), leukaemia (Konaka et al., Cell, 1984, 37, 1035) and ovarian, bronchial or pancreatic cancer (European Patent Specification No. 0400586). As further human tumour tissues are tested for receptor tyrosine kinase activity it is expected that its widespread prevalance will be established in further cancers such as thyroid and uterine cancer. It is also known that tyrosine kinase activity is rarely detected in normal cells whereas it is more frequently detectable in malignant cells (Hunter, Cell, 1987, 50, 823). It has been shown more recently (U.J. Gullick, Brit. Med. Bull., 1991, 47, 87) that epidermal growth factor receptor which possesses tyrosine kinase activity is overexpressed in many human cancers such as brain, lung squamous cell, bladder, gastric, breast, head and neck, oesophageal, gynaecological and thyroid tumours.

Accordingly it has been recognised that an inhibitor of receptor tyrosine kinase should be of value as a selective inhibitor of the growth of mammalian cancer cells (Yaish et al. Science, 1988, 242, 933). Support for this view is probided by the demonstration that erbstatin, a receptor tyrosine kinase inhibitor, specifically attenuates the growth of a human mammary carcinoma which expresses epidermal growth factor (EGF) receptor tyrosine kinase but is without effect on the growth of other carcinomas which do not express EGF receptor tyrosine kinase (Toi et al. Eur. J. Cancer Clin. Oncol., 1990, 26, 722.) Various derivatives of styrene are also stated to possess tyrosine kinase inhibitory properties (European Patent Application Nos. 0211363, 0304493 and 0322738) and to be of use as anti-tumour agents. Various known tyrosine kinase inhibitors are disclosed in a more recent review by T. R. Burke Jr. (Drugs of the Future, 1992, 17, 119).

We have now found that certain known quinazoline derivatives and also novel quinazoline derivatives possess anti-cancer properties which are believed to arise from their receptor tyrosine kinase inhibitory properties.

According to one aspect of the invention there is provided a pharmaceutical composition which comprises a quinazoline derivative of the formula I (set out hereinafter)

wherein $R^1$ is hydrogen, trifluoromethyl or nitro, n is 1 and $R^2$ is halogeno, trifluoromethyl, nitro, cyano, (1-4C)alkyl, (1-4C)alkoxy, N-(1-4C)alkylamino, N,N-di-[(1-4C)alkyl]amino, (1-4C)alkylthio, (1-4C)alkylsulphinyl or (1-4C)alkylsulphonyl, except that 7-trifluoromethyl-4-(4'-methoxyanilino)quinazoline or the pharmaceutically-acceptable salts thereof are excluded ;

or wherein $R^1$ is 5-chloro, 6-chloro, 6-bromo or 8-chloro, n is 1 and $R^2$ is 3'-chloro or 3'-methyl, except that 5-chloro-4-(3'-chloroanilino)-, 6-chloro-4-(3'-methylanilino)- and 8-chloro-4-(3'-chloroanilino)-quinazoline or the pharmaceutically-acceptable salts thereof are excluded;

or wherein $R^1$ is hydrogen, halogeno, trifluoromethyl or nitro, n is 2 and each $R^2$, which may be the same or different, is halogeno, (1-4C)alkyl or (1-4C)alkoxy, except that 6-fluoro-4-(2',4'-dimethylanilino)quinazoline or the pharmaceutically-acceptable salts thereof are excluded;

or a pharmaceutically-acceptable salt thereof; together with a pharmaceutically-acceptable diluent or carrier.

According to a further aspect of the invention there is provided a pharmaceutical composition which comprises a quinazoline derivative of the formula I wherein

$R^1$ is hydrogen, n is 1 and $R^2$ is halogeno, (1-4C)alkyl or (1-4C)alkoxy;
or $R^1$ is hydrogen, halogeno, trifluoromethyl or nitro, n is 2 and each $R^2$, which may be the same or different, is halogeno, (1-4C)alkyl or (1-4C)alkoxy, except that 6-fluoro-4-(2',4'-dimethylanilino)-quinazoline or the pharmaceutically-acceptable salts thereof are excluded;

or a pharmaceutically-acceptable salt thereof; together with a pharmaceutically-acceptable diluent or carrier.

The chemical formulae referred to herein by Roman numerals are set out for convenience on a separate sheet hereinafter. In this specification the term "alkyl" includes both straight and branched chain alkyl groups but references to individual alkyl groups such as "propyl" are specific for the straight chain version only. An analogous convention applies to other generic terms.

The quinazolines of the formula I are unsubstituted at the 2-position. This is specifically indicated in formula I by the hydrogen atom shown at the 2-position. It is to be understood that the $R^1$ group is located only on the benzo portion of the quinazoline ring.

Within the present invention it is to be understood that a quinazoline of the formula I may exhibit the phenomenon of tautomerism and that the formulae drawings within this specification can represent only one of the possible tautomeric forms. It is to be understood that the invention encompasses any tautomeric form which possesses anti-tumour activity and is not to be limited merely to any one tautomeric form utilised within the formulae drawings.

It is also to be understood that certain quinazolines of the formula I can exist in solvated as well as unsolvated forms such as, for example, hydrated forms. It is to be understood that the invention encompasses all such solvated forms which possess anti-tumour activity.

Suitable values for the generic radicals referred to above include those set out below.

A suitable value for $R^1$ or $R^2$ when it is halogeno is, for example, fluoro, chloro, bromo or iodo; for $R^2$ when it is (1-4C)alkyl is, for example, methyl, ethyl, propyl, isopropyl or butyl; for $R^2$ when it is (1-4C)alkoxy is, for example, methoxy, ethoxy, propoxy or isopropoxy; for $R^2$ when it is N-(1-4C)alkylamino, is, for example, N-methylamino, N-ethylamino or N-propylamino; for $R^2$ when it is N,N-di-[(1-4C)alkyl]amino is, for example, N,N-dimethylamino, N-ethyl-N-methylamino, N,N-diethylamino, N-methyl-N-propylamino or N,N-dipropylamino; for $R^2$ when it is (1-4C)alkylthio is, for example, methylthio, ethylthio or propylthio; for $R^2$ when it is (1-4C)alkylsulphinyl is, for example, methylsulphinyl, ethylsulphinyl or propylsulphinyl; and for $R^2$ when it is (1-4C)alkylsulphonyl is, for example, methylsulphonyl, ethylsulphonyl or propylsulphonyl.

A suitable pharmaceutically-acceptable salt of a quinazoline of the invention which is sufficiently basic is, for example, an acid-addition salt with, for example, an inorganic or organic acid, for example hydrochloric, hydrobromic, sulphuric, phosphoric, trifluoroacetic, citric, maleic, oxalic, fumaric or tartaric acid.

The composition may be in a form suitable for oral administration, for example, as a tablet or capsule, for parenteral injection (including intraveous, subcutaneous, intramuscular, intravascular or infusion) as a sterile solution, suspension or emulsion, for topical administration as an ointment or cream or for rectal administration as a suppository.

In general the above compositions may be prepared in a conventional manner using conventional excipients.

The quinazoline will normally be administered to a warm-blooded animal at a unit dose within the range 5-5000 mg per square metre body area of the animal, i.e. approximately 0.1-100 mg/kg, and this normally provides a therapeutically-effective dose. A unit dose form such as a tablet or capsule will usually contain, for example 1-250 mg of active ingredient. Preferably a daily dose in the range of 1-50 mg/kg is employed. However the daily dose will necessarily be varied depending upon the host treated, the particular route of administration, and the severity of the illness being treated. Accordingly the optimum dosage will be determined by the practitioner who is treating any particular patient.

Many quinazoline derivatives are already known and some are also known to possess pharmacological properties. It is believed however that the pharmaceutical compositions defined hereinbefore do not embrace any such pharmacologically-active quinazoline derivative.

It is known from UK Patent Application No. 2033894 that certain quinazoline derivatives possess analgesic and anti-inflammatory properties. The compounds, and pharmaceutical compositions containing them, are disclosed by way of a generic formula II wherein $R^1$ is hydrogen, halogeno, trifluoromethyl or nitro; $R^2$ is hydrogen, halogeno, alkyl or alkoxy; and $R^3$ is hydrogen or alkyl.

With one exception, all of the examples or named compounds therein require $R^1$ to be a substituent other than hydrogen. The exception is the compound 4-(N-methylanilino)quinazoline i.e. each of $R^1$ and $R^2$ is hydrogen and $R^3$ is methyl. It is believed that the pharmaceutical compositions containing quinazoline derivatives disclosed hereinbefore do not embrace compositions containing any of the specifically disclosed compounds of UK Patent Specification No.

2033894.

Further known quinazoline derivatives mentioned in UK 2033894 include the compounds 4-anilinoquinazoline and 4-anilino-6-chloroquinazoline [J. Org. Chem., 1976, 41, 2646 and US Patent No. 3985749 respectively], known for use in the treatment of coccidiosis.

It is known from Japanese Patent Specification No. 57144266 [Chemical Abstracts, volume 98, abstract number 89384x, and the Registry File entries associated therewith] that certain 4-anilino-6-fluoroquinazolines possess analgesic and anti-inflammatory properties. The only disubstituted anilino derivative disclosed therein is believed to be 6-fluoro-4-(2',4'-dimethylanilino)-quinazoline.

It is further known from Chemical Abstracts, volume 96, abstract number 122727v, and the Registry File entries associated therewith, that certain 4-(3'-aminomethyl-4'-hydroxyanilino)-quinazolines possess antiarrhthymic properties. Compounds mentioned as intermediates include 4-(2'-chloroanilino)-, 4-(2',4'-dichloro-anilino)- and 4-(4'-bromoanilino)-quinazoline. It is known from Chemical Abstracts, volume 100, abstract number 34492k, and the Registry File entries associated therewith, that certain 4-substituted quinazolines were tested for herbicidal, insecticidal, acaricidal and fungicidal activity. Only 4-chloroquinazoline is stated to possess any activity. Compounds disclosed included 4-(3'-chloroanilino)- and 4-(3',4'-dimethylanilino)quinazoline. Further known quinazoline derivatives which are not stated to possess pharmacological properties are 4-(4'-chloroanilino)- and 4-(2',6'-dimethylanilino)-quinazoline [Chem. Abs., 107, 198230u] and 4-(4'-ethylanilino)- and 4-(4'-methoxyanilino)quinazoline [Chem. Abs., 76, 34199f].

According to a further aspect of the present invention there is provided a quinazoline derivative of the formula I as defined hereinbefore for use in a method of treatment of the human or animal body by therapy.

Certain of the quinazoline derivatives of the formula I are novel and this provides a further aspect of the present invention. According to this aspect there is provided a quinazoline derivative of the formula I

wherein $R^1$ is hydrogen, n is 1 and $R^2$ is 2'-methoxy, 3'-methoxy, 3'-fluoro, 3'-bromo, 3'-iodo, 3'-ethyl, 3'-nitro, 3'-cyano, 3'-methylthio or 3'-(N,N-dimethylamino);
or wherein $R^1$ is 5-chloro, 6-chloro, 8-chloro, 6-bromo or 7-nitro, n is 1 and $R^2$ is 3'-chloro or 3'-methyl, except that 5-chloro-4-(3'-chloroanilino)-, 6-chloro-4-(3'-methylanilino)- and 8-chloro-4-(3'-chloroanilino)-quinazoline or the pharmaceutically-acceptable salts thereof are excluded;
or wherein $R^1$ is hydrogen or chloro, n is 2 and each $R^2$, which may be the same or different, is chloro, methyl or methoxy, except that 4-(2',4'-dichloroanilino)-, 4-(2',6'-dimethylanilino)- and 4-(3',4'-dimethylanilino)-quinazoline or the pharmaceutically-acceptable salts thereof are excluded;

or a pharmaceutically-acceptable salt thereof.

According to a further aspect of the invention there is provided a quinazoline derivative of the formula I wherein $R^1$ is 5-chloro, 6-chloro, 8-chloro, 6-bromo, 6-nitro or 7-nitro, n is 1 and $R^2$ is 3'-chloro or 3'-methyl, except that 5-chloro-4-(3'-chloroanilino)-, 6-chloro-4-(3'-methylanilino)- and 8-chloro-4-(3'-chloroanilino)-quinazoline or the pharmaceutically-acceptable salts thereof are excluded; or a pharmaceutically-acceptable salt thereof.

According to a further aspect of the invention there is provided a quinazoline derivative of the formula I wherein $R^1$ is hydrogen, n is 1 and $R^2$ is 2'- or 3'-methoxy; or $R^1$ is hydrogen or chloro, n is 2 and each $R^2$, which may be the same or different, is chloro, methyl or methoxy, except that 4-(2',4'-dichloroanilino)-, 4-(2',6'-dimethylanilino)- and 4-(3',4'-dimethylanilino)-quinazoline or the pharmaceutically-acceptable salts thereof are excluded; or a pharmaceutically-acceptable salt thereof.

A specific preferred novel compound of the invention is:-
4-(3'-methoxyanilino)quinazoline or 7-chloro-4-(4'-chloro-2'-methylanilino)quinazoline; or a pharmaceutically-acceptable acid-addition salt thereof.

Further specific preferred novel compounds of the invention are:-

4-(3'-bromoanilino)quinazoline,
4-(3'-iodoanilino)quinazoline,
6-chloro-4-(3'-chloroanilino)quinazoline,
6-bromo-4-(3′-methylanilino)quinazoline and
4-(3′-nitroanilino)quinazoline;

or a pharmaceutically-acceptable acid-addition salt thereof.

We have now found that many of these known compounds and the novel compounds of the invention possess anti-cancer properties which are believed to arise from their receptor tyrosine kinase inhibitory properties.

Thus according to this aspect of the invention there is probided the use of the quinazoline derivatives of the formula I, or a pharmaceutically-acceptable salt thereof,
wherein $R^1$ is hydrogen, halogeno, trifluoromethyl or nitro, n is 1 or 2 and each $R^2$, which may be the same or different,

is hydrogen, halogeno, trifluoromethyl, nitro, cyano, amino, (1-4C)alkyl, (1-4C)alkoxy, N-(1-4C)alkylamino, N,N-di-[(1-4C)alkyl]amino, (1-4C)alkylthio, (1-4C)alkylsulphinyl or (1-4C)alkylsulphonyl, in the manufacture of a medicament for use in the production of an anti-cancer effect in a warm-blooded animal such as man.

According to a further aspect of the present invention there is probided the use of the quinazoline derivatives of the formula I, or a pharmaceutically-acceptable salt thereof,
wherein $R^1$ is hydrogen, halogeno, trifluoromethyl or nitro, n is 1 or 2 and each $R^2$, which may be the same or different, is hydrogen, halogeno, (1-4C)alkyl or (1-4C)alkoxy, in the manufacture of a medicament for use in the production of an anti-cancer effect in a warm-blooded animal such as man.

Suitable values for the generic radicals referred to above include those set out hereinbefore.

According to a further feature of this aspect of the invention there is probided a method for producing an anti-cancer effect in a warm-blooded animal, such as man, in need of such treatment which comprises administering to said animal an effective amount of a quinazoline derivative as defined in the paragraphs immediately above.

Particular groups of compounds of the invention for use in the manufacture of medicaments as defined before, or for use in a method of treatment as defined hereinbefore, include, for example, those compounds of the formula I, or pharmaceutically-acceptable salts thereof, wherein:-

(a) $R^1$ is hydrogen, fluoro or chloro, n is 1 or 2 and each $R^2$, which may be the same or different, is hydrogen, fluoro, chloro, bromo, methyl, ethyl, methoxy or ethoxy;

(b) $R^1$ is hydrogen or chloro, n is 1 or 2 and each $R^2$, which may be the same or different, is chloro, bromo, methyl or methoxy;

(c) $R^1$ is hydrogen, n is 1 and $R^2$ is chloro, methyl or methoxy;

(d) $R^1$ is hydrogen or chloro, n is 2 and each $R^2$, which may be the same or different, is chloro or methyl;

(e) 4-(3'-methylanilino)quinazoline or 4-(3'-chloroanilino)-quinazoline;

(f) $R^1$ is hydrogen, fluoro, chloro, bromo or nitro, n is 1 or 2 and each $R^2$, which may be the same or different, is hydrogen, fluoro, chloro, bromo, iodo, trifluoromethyl, nitro, cyano, methyl, ethyl, methoxy, ethoxy, methylthio or N,N-dimethylamino;

(g) $R^1$ is hydrogen, chloro or bromo, n is 1 and $R^2$ is chloro, bromo, iodo, nitro, methyl or methoxy;

(h) $R^1$ is hydrogen, 6-chloro, 7-chloro or 6-bromo, n is 1 and $R^2$ is 3'-chloro, 3'-bromo, 3'-iodo, 3'-nitro, 3'-methyl or 3'-methoxy;

(i) 4-(3'-bromoanilino)quinazoline, 4-(3'-iodoanilino)-quinazoline, 6-bromo-4-(3'-methylanilino)quinazoline or 7-chloro-4-(3'-chloroanilino)quinazoline.

The anti-cancer treatment defined hereinbefore may be applied as a sole therapy or may involve, in addition to the quinazoline derivative of the invention, one or more other anti-tumour substances, for example those selected from, for example, mitotic inhibitors, for example vinblastine; alkylating agents, for example cis-platin, carboplatin and cyclophosphamide; antimetabolites, for example 5-fluorouracil, cytosine arabinoside and hydroxyurea, or, for example, one of the preferred antimetabolites disclosed in European Patent Application No. 239362 such as N-{5-[N-(3,4-dihydro-2-methyl-4-oxoquinazolin-6-ylmethyl)-N-methylamino]-2-thenoyl}-L-glutamic acid; intercalating antibiotics, for example adriamycin and bleomycin; enzymes, for example asparaginase; topoisomerase inhibitors, for example etoposide; biological response modifiers, for example interferon; and anti-hormones, for example antioestrogens such as 'NOLVADEX' (tamoxifen) or, for example antiandrogens such as 'CASODEX' (4′-cyano-3-(4-fluorophenylsulphonyl)-2-hydroxy-2-methyl-3′-(trifluoromethyl)propionanilide. Such conjoint treatment may be achieved by way of the simultaneous, sequential or separate dosing of the individual components of the treatment. According to this aspect of the invention there is probided a pharmaceutical product comprising a quinazoline derivative of the formula I as defined hereinbefore and an additional anti-tumour substance as defined hereinbefore for the conjoint treatment of cancer.

As stated above the quinazoline derivative defined in the present invention is an effective anti-cancer agent, which property is believed to arise from its receptor kinase inhibitory properties. Such a quinazoline derivative of the invention is expected to possess a wide range of anti-cancer properties as receptor tyrosine kinases have been implicated in many common human cancers such as leukaemia and breast, lung, colon, rectal, stomach, prostate, bladder, pancreas and ovarian cancer. Thus it is expected that a quinazoline derivative of the invention will possess anti-cancer activity

against these cancers. It is in addition expected that a quinazoline of the present invention will possess activity against a range of leukaemias, lymphoid malignancies and solid tumours such as carcinomas and sarcomas in tissues as the liver, kidney, prostate and pancreas.

A novel quinazoline derivative of the formula I, or a pharmaceutically-acceptable salt thereof, may be prepared by any process known to be applicable to the preparation of chemically-related compounds. A suitable process is, for example, illustrated by that used in UK Patent Application No. 2033894. Such a process, when used to prepare a novel quinazoline derivative of the formula I, or a pharmaceutically-acceptable salt thereof, is probided as a further feature of the invention and is illustrated by the following representative example in which, unless otherwise stated, $R^1$, n and $R^2$ have any of the meanings defined hereinbefore for a novel quinazoline derivative of the formula I.

The reaction, preferably in the presence of a suitable base, of a quinazoline of the formula III (set out hereinafter), wherein Z is a displaceable group, with an aniline of the formula IV.

A suitable displaceable group Z is, for example, a halogeno or sulphonyloxy group, for example a chloro, bromo, methanesulphonyloxy or toluene-p-sulphonyloxy group.

A suitable base is, for example, an organic amine base such as, for example, pyridine, 2,6-lutidine, collidine, 4-dimethylaminopyridine, triethylamine, morpholine or diazabicyclo[5.4.0]undec-7-ene, or, for example, an alkali or alkaline earth metal carbonate or hydroxide, for example sodium carbonate, potassium carbonate, calcium carbonate, sodium hydroxide or potassium hydroxide.

The reaction is preferably carried out in the presence of a suitable inert solvent or diluent, for example a (1-4C) alkanol such as methanol, ethanol or isopropanol, a halogenated solvent such as methylene chloride, chloroform or carbon tetrachloride, an ether such as tetrahydrofuran or 1,4-dioxan, an aromatic solvent such as toluene, or a dipolar aprotic solvent such as N,N-dimethylformamide, N,N-dimethylacetamide, N-methylpyrrolidin-2-one or dimethylsulphoxide. The reaction is conveniently carried out at a temperature in the range, for example, 10 to 150°C, preferably in the range 20-80°C.

The quinazoline derivative of the formula I may be obtained from this process in the form of the free base or alternatively it may be obtained in the form of a salt with the acid of the formula H-Z wherein Z has the meaning defined hereinbefore. When it is desired to obtained the free base from the salt, the salt may be treated with a suitable base as defined hereinbefore using a conventional procedure.

When a pharmaceutically-acceptable salt of a novel quinazoline derivative of the formula I is required, it may be obtained, for example, by reaction of said compound with, for example, a suitable acid using a conventional procedure.

As stated hereinbefore the quinazoline derivative defined in the present invention possesses anti-cancer activity which is believed to arise from the receptor tyrosine kinase inhibitory activity of the compound. These properties may be assessed, for example, using one or more of the procedures set out below:-

(a) An in vitro assay which determines the ability of a test compound to inhibit the enzyme receptor tyrosine kinase. Receptor tyrosine kinase was obtained in partially purified form from A-431 cells (derived from human vulval carcinoma) by procedures related to those described by Carpenter et al., J. Biol. Chem., 1979, 254, 4884, Cohen et al., J. Biol. Chem., 1982, 257, 1523 and by Braun et al., J. Biol. Chem., 1984, 259, 2051.

A-431 cells were grown to confluence using Dulbecco's modified Eagle's medium (DMEM) containing 5% fetal calf serum (FCS). The obtained cells were homogenised in a hypotonic borate/EDTA buffer at pH 10.1. The homogenate was centrifuged at 400 g for 10 minutes at 0-4°C. The supernatant was centrifuged at 25,000 g for 30 minutes at 0-4°C. The pelleted material was suspended in 30 mM Hepes buffer at pH 7.4 containing 5% glycerol, 4 mM benzamidine and 1% Triton X-100, stirred for 1 hour at 0-4°C, and recentrifuged at 100,000 g for 1 hour at 0-4°C. The supernatant, containing solubilised receptor tyrosine kinase, was stored in liquid nitrogen.

For test purposes 40 µl of the enzyme solution so obtained was added to a mixture of 400 µl of a mixture of 150 mM Hepes buffer at pH 7.4, 500 µM sodium orthovanadate, 0.1% Triton X-100, 10% glycerol, 200 µl water, 80 µl of 25 mM DTT and 80µl of a mixture of 12.5 mM manganese chloride, 125 mM magnesium chloride and distilled water. There was thus obtained the test enzyme solution.

Each test compound was dissolved in dimethylsulphoxide (DMSO) to give a 50 mM solution which was diluted with 40 mM Hepes buffer containing 0.1% Triton X-100, 10% glycerol and 10% DMSO to give a 500 µM solution. Equal volumes of this solution and a solution of epidermal growth factor (EGF; 20 µg/ml) were mixed.

[$\gamma$-$^{32}$P]ATP (3000 Ci/mM, 250 µCi) was diluted to a volume of 2 ml by the addition of a solution of ATP (100 µM) in distilled water. An equal volume of a 4 mg/ml solution of the peptide Arg-Arg-Leu-Ile-Glu-Asp-Ala-Glu-Tyr-Ala-Ala-Arg-Gly in a mixture of 40 mM Hepes buffer at pH 7.4, 0.1% Triton X-100 and 10% glycerol was added.

The test compound/EGF mixture solution (5 µl) was added to the test enzyme solution (10 µl) and the mixture was incubated at 0-4°C for 30 minutes. The ATP/peptide mixture (10 µl) was added and the mixture was incubated at 25°C for 10 minutes. The phosphorylation reaction was terminated by the addition of 5% trichloroacetic acid (40 µl) and bovine serum albumin (BSA; 1 mg/ml, 5 µl). The mixture was allowed to stand at 4°C for 30 minutes and then centrifuged. An aliquot (40 µl) of the supernatant was placed onto a strip of Whatman p 81 phosphocellulose paper. The strip was washed in 75 mM phosphoric acid (4 x 10 ml) and blotted dry. Radioactivity present in the filter paper was

measured using a liquid scintillation counter (Sequence A). The reaction sequence was repeated in the absence of the EGF (Sequence B) and again in the absence of the test compound (Sequence C).

Receptor tyrosine kinase inhibition was calculated as follows:-

$$\% \text{ Inhibition} = \frac{100 - (A\text{-}B)}{C - B} \times 100$$

The extent of inhibition was then determined at a range of concentrations of test compound to give an $IC_{50}$ value.

(b) An in vitro assay which determines the ability of a test compound to inhibit the growth of the human nasopharyngeal cell line KB.

KB cells were seeded into wells at a density of 1 x 10 - 1.5 x $10^4$ cells per well and grown for 24 hours in DMEM supplemented with 5% FCS (charcoal-stripped). Cell growth was determined after incubation for 3 days by the extent of metabolism of MTT tetrazolium dye to furnish a bluish colour. Cell growth was then determined in the presence of EGF (10 ng/ml) or in the presence of EGF (10 ng/ml) and a test compound at a range of concentrations. An $IC_{50}$ value could then be calculated.

Although the pharmacological properties of the compounds of the formula I vary with structural change as expected, in general activity possessed by compounds of the formula I may be demonstrated at the following concentrations in one or both of the above tests (a) and (b):-

Test (a):- $IC_{50}$ in the range, for example, 0.01-10 μM;
Test (b):- $IC_{50}$ in the range, for example, 0.1-100 μM.

Thus, by way of example, the compound 4-(3'-methylanilino)-quinazoline has an $IC_{50}$ of 0.18 μM in Test (a) and an $IC_{50}$ of approximately 5 μM in Test (b); the compound 4-(3'-chloroanilino)quinazoline has an $IC_{50}$ of 0.04 μM in Test (a) and an $IC_{50}$ of approximately 5 μM in Test (b); the compound 4-(3'-bromoanilino)quinazoline has an $IC_{50}$ of 0.02 μM in Test (a) and an $IC_{50}$ of 0.78 μM in Test (b); and the compound 7-chloro-4-(3'-chloroanilino)quinazoline has an $IC_{50}$ of 0.02 μM in Test (a) and an $IC_{50}$ of 1.0 μM in Test (b).

The invention will now be illustrated in the following non-limiting Examples in which, unless otherwise stated:-

(i) evaporations were carried out by rotary evaporation in vacuo and work-up procedures were carried out after removal of residual solids such as drying agents by filtration;
(ii) yields are given for illustration only and are not necessarily the maximum attainable;
(iii) melting points are uncorrected and were determined using a Mettler SP62 automatic melting point apparatus, an oil-bath apparatus or a Koffler hot plate apparatus.

**Example 1**

3-Chloroaniline (7.3 g) was added to a mixture of 4-chloroquinazoline (9 g), triethylamine (6.2 ml) and methylene chloride (90 ml). The mixture was stirred at ambient temperature for 30 minutes and evaporated. The residue was dissolved in ethanol (250 ml) and the solution was heated to reflux for 30 mintues. The mixture was allowed to cool to ambient temperature and to stand for 4 hours. The precipitate was filtered off and washed with cold ethanol. There was thus obtained 4-(3′-chloroanilino)quinazoline hydrochloride (11 g, 68%), m.p. 218-225°C.

Elemental Analysis: Found C, 57.4; H, 3.8; N, 14.1;
$C_{14}H_{11}Cl_2N_3$ requires C, 57.6; H, 3.8; N, 14.4%.

**Example 2**

The procedure described in Example 1 was repeated except that the appropriate aniline was used in place of 3-chloroaniline. There were thus obtained, as hydrochloride salts, the compounds described in the following table, the structures of which were confirmed by elemental analysis.

**TABLE I**

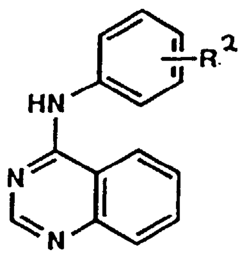

| Example 2 Compound No. | $R^2$ | m.p. (°C) |
|---|---|---|
| 1 | 3'-methyl | 198-205 |
| 2 | 4'-chloro | 210-212 |
| 3 | 4'-bromo | 219-221 |
| 4 | 3'-methoxy | 216-218 |

## Example 3

A mixture of 4-chloroquinazoline (1.65 g) and 2,5-dimethylaniline (2.42 g) was heated to 100°C for 3 days. The mixture was cooled to ambient temperature and the residue was partitioned between methylene chloride and water. The organic layer was dried ($MgSO_4$) and evaporated. The residue was purified by column chromatography using increasingly polar mixtures of methylene chloride and methanol as eluent. There was thus obtained 4-(2',5'-dimethylanilino)quinazoline (2.2 g), m.p. 112-113°C.

Elemental Analysis: Found C, 76.9; H, 6.1; N, 16.7;
$C_{16}H_{15}N_3$ requires C, 77.1; H, 6.0; N, 16.9%.

## Example 4

A mixture of 4,7-dichloroquinazoline (1.89 g) and 4-chloro-2-methylaniline (1.40 g) was heated to 100°C for 5 minutes. The mixture was observed to melt and then resolidify. Ethanol (5 ml) was added and the mixture was heated to 100°C for 30 minutes. The mixture was cooled to ambient temperature and the solid was isolated. There was thus obtained 7-chloro-4-(4'-chloro-2'-methylanilino)-quinazoline hydrochloride (1.5 g), m.p. 275-280°C (recrystallised from ethanol).

Elemental Analysis: Found C, 52.5; H, 3.7; N, 12.2;
$C_{15}H_{12}ClN_3$ requires C, 52.9; H, 3.5; N, 12.3%.

The 4,7-dichloroquinazoline used as a starting material was obtained as follows:-
A mixture of 4-chloroanthranilic acid (17.2 g) and formamide (10 ml) was stirred and heated to 130°C for 45 minutes

and to 175°C for 75 minutes. The mixture was allowed to cool to approximately 100°C and 2-(2-ethoxyethoxy)ethanol (50 ml) was added. The solution so formed was poured into a mixture (250 ml) of ice and water. The precipitate was isolated, washed with water and dried. There was thus obtained 7-chloroquinazolin-4-one (15.3 g, 85%).

Phosphoryl chloride (8.35 ml) was added dropwise to a stirred mixture of 7-chloroquinazolin-4-one (8.09 g), N,N-dimethylaniline (9.77 g) and toluene (140 ml) and the mixture was heated to reflux for 4 hours. The mixture was evaporated and the residue was purified by column chromatography using increasingly polar mixtures of methylene chloride and ethyl acetate as eluent. There was thus obtained the required starting material (4.72 g, 53%).

**Example 5**

The procedure described in Example 1 was repeated except that the appropriate aniline was used in place of 3-chloroaniline and, where appropriate, the appropriate substituted 4-chloroquinazoline was used in place of 4-chloroquinazoline. There were thus obtained, as hydrochloride salts, the compounds described in the following table, the structures of which were confirmed by elemental analysis.

**TABLE II**

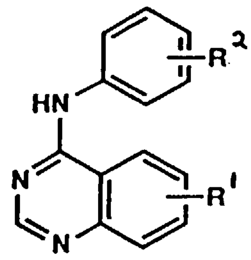

| Example 5 Compd. No. | $R^1$ | $R^2$ | m.p. (°C) |
|---|---|---|---|
| 1 | H | 3'-fluoro | 258-260 |
| 2[a] | 6-chloro | 3'-chloro | 215-217 |
| 3[b] | 6-chloro | 3'-methyl | 213-214 |
| 4 | 7-chloro | 3'-chloro | 274-275 |
| 5[c] | 7-chloro | 3'-methyl | 221-222 |
| 6[d] | 5-chloro | 3'-chloro | 229-233 |
| 7[d] | 5-chloro | 3'-methyl | 227-230 |
| 8[e] | 8-chloro | 3'-chloro | 237-239 |
| 9 | 8-chloro | 3'-methyl | 222 |
| 10[f] | H | 3'-(N,N-dimethylamino) | 176-178 |

**Notes**

a. The 4,6-dichloroquinazoline used as a starting material was obtained as follows:-

A mixture of 5-chloroanthranilic acid (17.2 g) and formamide (10 ml) was stirred and heated to 130°C for 45 minutes and to 175°C for 75 minutes. The mixture was allowed to cool to approximately 100°C and 2-(2-ethoxyethoxy)ethanol (50 ml) was added. The solution so formed was poured into a mixture (250 ml) of ice and water. The precipitate was isolated, washed with water and dried. There was thus obtained 6-chloroquinazolin-4-one (17.45 g, 96%).

Phosphoryl chloride (6.84 ml) was added dropwise to a stirred suspension of 6-chloroquinazolin-4-one (6.63 g), N,N-dimethylaniline (8.01 g) and toluene (100 ml) and the mixture was stirred and heated to reflux for 5 hours. The mixture was cooled to ambient temperature and poured into a saturated aqueous ammonium hydroxide solution. The organic layer was washed with water, dried ($MgSO_4$) and evaporated. The residue was purified by column chromatography using initially methylene chloride and then increasingly polar mixtures of methylene chloride and ethyl acetate as eluent. There was thus obtained the required starting material (5.34 g, 75%).

b. The product contained only 0.55 equivalents of hydrogen chloride.

c. The product contained only 0.05 equivalents of hydrogen chloride.

d. The addition of diethyl ether to the ethanolic solution led to the precipitation of the product.

The 4,5-dichloroquinazoline used as a starting material was obtained from 6-chloroanthranilic acid using analogous procedures to those described in Note a. above.

e. The 4,8-dichloroquinazoline used as starting material was

obtained from 3-chloroanthranilic acid using analogous procedures to those described in Note a. above.

f. No precipitate was deposited from the ethanolic solution. The solution was evaporated and the product was purified by column chromatography using increasingly polar mixtures of methylene chloride and ethyl acetate as eluent. There was thus obtained 4-[3'-(N,N-dimethylamino)anilino]quinazoline in 60% yield.

## Example 6

A mixture of 4-chloroquinazoline (0.5 g) and 2-chloroaniline (1 ml) was stirred and heated to 80°C for 10 minutes. The solid mixture was cooled to ambient temperature and recrystallised from isopropanol. There was thus obtained 4-(2'-chloroanilino)quinazoline (0.57 g), m.p. 225-227.5°C.

Elemental Analysis: Found C, 57.5; H, 4.5; N, 13.0;
$C_{14}H_{10}ClN_3$. 1.2HCl. 0.5$(CH_3)_2$CHOH requires C, 57.3; H, 4.7; N, 12.9%.

## Example 7

3-Bromoaniline (0.52 g) was added to a stirred solution of 4-chloroquinazoline (0.5 g) in isopropanol (10 ml) which had been heated to 80°C and the mixture was stirred at 80°C for 30 minutes. The mixture was cooled to ambient temperature and the precipitate was filtered off and washed with cold isopropanol. There was thus obtained 4-(3'-bromoanilino)quinazoline hydrochloride (0.61 g, 61%), m.p. 252-256°C.

Elemental Analysis: Found C, 49.9; H, 3.2; N, 12.4;
$C_{14}H_{10}BrN_3$. HCl requires C, 49.9; H, 3.3.; N, 12.5%.

## Example 8

The procedure described in Example 7 was repeated except that the appropriate aniline was used in place of 3-bromoaniline and, where appropriate, the appropriate substituted 4-chloroquinazoline was used in place of 4-chloroquinazoline. There were thus obtained, as hydrochloride salts, the compounds described in the following table, the structures of which were confirmed by elemental analysis.

TABLE III

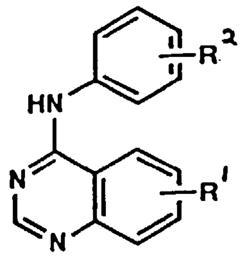

| Example 8 Compd. No. | $R^1$ | $R^2$ | m.p. (°C) |
|---|---|---|---|
| 1 | H | 3'-iodo | 243-246 |
| 2 | H | 3'-ethyl | 210-212 |
| 3[a] | 7-nitro | 3'-chloro | 234-236 |
| 4[b] | 7-nitro | 3'-methyl | >200 (decomposes) |
| 5 | H | 3'-trifluoromethyl | 211-212 |
| 6[c] | H | 3'-nitro | >280 |
| 7[d] | H | 3'-methylthio | 207-210 |

**Notes**

a. The product contained 1.0 equivalent of hydrogen chloride and 0.9 equivalents of isopropanol.

The 4-chloro-7-nitroquinazoline used as a starting material

was obtained from 4-nitroanthranilic acid using analogous procedures to those described in Note a. below Table II in Example 5 except that the 7-nitroquinazolin-4-one intermediate was converted into 4-chloro-7-nitroquinazoline by treatment with thionyl chloride, containing one drop of dimethylformamide, at reflux for 2 hours and by evaporation of the resultant solution.

b. The reactants were not heated to 80°C but were merely stirred at ambient tempeature for 20 minutes. The precipitated product was isolated and washed in turn with isopropanol and diethyl ether. The product gave the following analytical data: Found C, 57.4; H, 4.8; N, 16.3;
$C_{15}H_{12}N_4O_2$. HCl. 0.3$(CH_3)_2$CHOH requires C, 57.0; H, 4.6; N, 16.7%.

c. The product gave the following analytical data: Found C, 55.5; H, 3.6; N, 18.3;
$C_{14}H_{10}N_4O_2$. HCl requires C, 55.5; H, 3.6; N, 18.5%.

d. Diethyl ether was added to the isopropanol solution to precipitate the product.

**Example 9**

A mixture of 6-bromo-4-chloroquinazoline (2 g), 3-methylaniline (0.88 g) and isopropanol (30 ml) was stirred and heated to reflux for 1 hour. The mixture was cooled to ambient temperature. The precipitate was filtered off and washed with cold isopropanol and with diethyl ether. There was thus obtained 6-bromo-4-(3'-methylanilino)quinazoline hydrochloride (1.36 g, 47%), m.p. 245-251°C.

Elemental Analysis: Found C, 50.4; H, 3.6; N, 12.0;
$C_{15}H_{12}BrN_3$. 1.18HCl requires C, 50.4; H, 3.7; N, 11.8%.

The 6-bromo-4-chloroquinazoline used as a starting material was obtained as follows:-

A mixture of 5-bromoanthranilic acid (15.2 g) and formamide (20 ml) was heated to 140°C for 2 hours and then to 190°C for 2 hours. The mixture was cooled to ambient temperature. Methanol (20 ml) was added and the mixture was heated to reflux for 5 minutes. Water (150 ml) was added and the mixture was cooled to ambient temperature. The precipitate was washed with water and dried. There was thus obtained 6-bromoquinazolin-4-one (14.1 g).

N,N-Dimethylaniline (7.77 g) and phosphoryl chloride (10.9 g) were added in turn to a stirred mixture of 6-bromoquinazolin-4-one (8 g) and toluene (80 ml) and the mixture was heated to reflux for 30 minutes. The mixture was cooled to ambient temperature and allowed to stand for 16 hours. The precipitate was isolated to give the required starting material (5.7 g).

**Example 10**

Using an analogous procedure to that described in Example 9, 4-chloroquinazoline was reacted with 3-cyanoaniline to give 4-(3'-cyanoanilino)quinazoline hydrochloride in 88% yield, m.p. >280°C.

Elemental Analysis: Found C, 64.0; H, 3.9; N, 19.9;
$C_{15}H_{10}N_4$. HCl requires C, 63.6; H, 3.9; N, 19.8%.

**Example 11**

3,5-Dichloroaniline (0.364 g) was added to a stirred mixture of 4-chloroquinazoline (0.358 g) and isopropanol (10 ml) and the mixture was stirred at ambient temperature for 5 minutes. The precipitate was isolated and washed in turn with isopropanol and diethyl ether. There was thus obtained 4-(3′,5′-dichloroanilino)quinazoline hydrochloride (0.556 g, 78%), m.p. >290°C.

Mass Spectrum: P m/e 290.
Elemental Analysis: Found C, 51.3; H, 3.1; N, 12.9;
$C_{14}H_9Cl_2N_3$. 1HCl requires C, 51.5; H, 3.1; N, 12.8%.

**Example 12**

Using an analogous procedure to that described in Example 11, 4-chloroquinazoline was reacted with 3,5-dimethylaniline to give 4-(3′,5′-dimethylanilino)quinazoline hydrochloride in 49% yield, m.p. 285-288°C.

Mass Spectrum: (P+1) m/e 250.
Elemental Analysis: Found C, 66.8; H, 5.8; N, 14.4;
$C_{16}H_{15}N_3$. 1HCl requires C, 67.2; H, 5.6; N, 14.7%.

**Example 13**

3-Methylaniline (0.139 g) was added to a mixture of 4-chloro-6-nitroquinazoline (0.25 g) and isopropanol (5 ml) and the mixture was stirred and heated to reflux for 2 hours. The mixture was cooled to ambient temperature and evaporated. The residue was purified by column chromatography using increasingly polar mixtures of hexane and ethyl acetate as eluent. There was thus obtained an oil which solidified on trituration under a mixture of diethyl ether and isopropanol. There was thus obtained 4-(3′-methylanilino)-6-nitroquinazoline (0.09 g, 26%), m.p. 248-249°C.

Mass Spectrum: (P+1) m/e 281.
Elemental Analysis: Found C, 64.0; H, 4.5; N, 18.6;
$C_{15}H_{12}N_4O_2$. $0.25(CH_3)_2CHOH$ requires C, 64.1; H, 4.8; N, 18.9%.

The 4-chloro-6-nitroquinazoline used as a starting material was obtained as follows:-

5-Nitroanthranilic acid was reacted with formamide using an analogous procedure to that described in Note a. below Table II in Example 5 for the corresponding reaction of 5-chloroanthranilic acid. There was thus obtained 6-nitroquinazolin-4-one in 82% yield, m.p. 268-271°C.

A mixture of 6-nitroquinazolin-4-one (10 g), phosphorus pentachloride (16.4 g) and phosphoryl chloride (20 ml) was heated to reflux for 2 hours. The mixture was cooled to ambient temperature and hexane (700 ml) was added. The mixture was stored at 0°C for 16 hours. The precipitate was isolated and partitioned between chloroform (700 ml) and water (500 ml). The aqueous layer was basified by the addition of 2N aqueous sodium hydroxide solution and extracted with chloroform (2 x 200 ml). The combined organic solutions were dried ($MgSO_4$) and evaporated. There was thus obtained the required starting material (1.6 g) which was used without further purification.

CHEMICAL FORMULAE

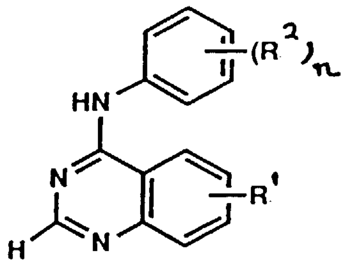

I

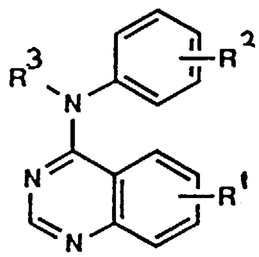

II

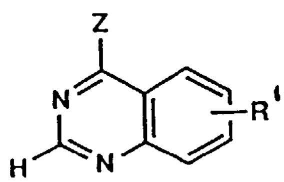

III

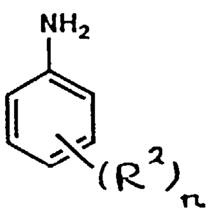

IV

**Claims**

**Claims for the following Contracting States : AT, BE, CH, DE, DK, FR, GB, IT, LI, LU, MC, NL, PT, SE**

1. A pharmaceutical composition which comprises a quinazoline derivative of the formula I

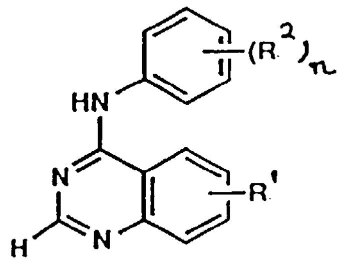

I

wherein $R^1$ is hydrogen, trifluoromethyl or nitro, n is 1 and $R^2$ is halogeno, trifluoromethyl, nitro, cyano, (1-4C) alkyl, (1-4C)alkoxy, N-(1-4C)alkylamino, N,N-di-[(1-4C)alkyl]amino, (1-4C)alkylthio, (1-4C)alkylsulphinyl or (1-4C)alkylsulphonyl, except that 7-trifluoromethyl-4-(4'-methoxyanilino)quinazoline or the pharmaceutically-acceptable salts thereof are excluded;
or wherein $R^1$ is 5-chloro, 6-chloro, 6-bromo or 8-chloro, n is 1 and $R^2$ is 3'-chloro or 3'-methyl, except that 5-chloro-4-(3'-chloroanilino)-, 6-chloro-4-(3'-methylanilino)- and 8-chloro-4-(3'-chloroanilino)-quinazoline or the pharmaceutically-acceptable salts thereof are excluded;
or wherein $R^1$ is hydrogen, halogeno, trifluoromethyl or nitro, n is 2 and each $R^2$, which may be the same or different, is halogeno, (1-4C)alkyl or (1-4C)alkoxy, except that 6-fluoro-4-(2',4'-dimethylanilino)quinazoline or the pharmaceutically-acceptable salts thereof are excluded;

or a pharmaceutically-acceptable salt thereof; together with a pharmaceutically-acceptable diluent or carrier.

2. A pharmaceutical composition which comprises a quinazoline derivative of the formula I as defined in claim 1 wherein

$R^1$ is hydrogen, n is 1 and $R^2$ is halogeno, (1-4C)alkyl or (1-4C)alkoxy;
or $R^1$ is hydrogen, halogeno, trifluoromethyl or nitro, n is 2 and each $R^2$, which may be the same or different, is halogeno, (1-4C)alkyl or (1-4C)alkoxy, except that 6-fluoro-4-(2',4'-dimethylanilino) quinazoline or the pharmaceutically-acceptable salts thereof are excluded;

or a pharmaceutically-acceptable salt thereof; together with a pharmaceutically-acceptable diluent or carrier.

3. A quinazoline derivative of the formula I

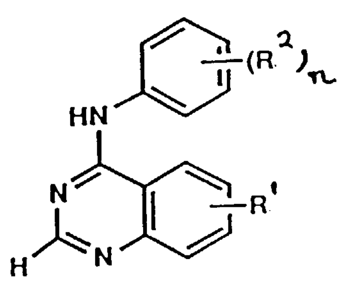

I

wherein $R^1$ is hydrogen, n is 1 and $R^2$ is 2'-methoxy, 3'-methoxy, 3'-fluoro, 3'-bromo, 3'-iodo, 3'-ethyl, 3'-nitro, 3'-cyano, 3'-methylthio or 3'-(N,N-dimethylamino);
or wherein $R^1$ is 5-chloro, 6-chloro, 8-chloro, 6-bromo or 7-nitro, n is 1 and $R^2$ is 3'-chloro or 3'-methyl, except that 5-chloro-4-(3'-chloroanilino)-, 6-chloro-4-(3'-methylanilino)- and 8-chloro-4-(3'-chloroanilino)-quinazoline

or the pharmaceutically-acceptable salts thereof are excluded;
or wherein $R^1$ is hydrogen or chloro, n is 2 and each $R^2$, which may be the same or different, is chloro, methyl or methoxy, except that 4-(2',4'-dichloroanilino)-, 4-(2',6'-dimethylanilino)- and 4-(3',4'-dimethylanilino)-quinazoline or the pharmaceutically-acceptable salts thereof are excluded;

or a pharmaceutically-acceptable salt thereof.

**4.** A quinazoline derivative of the formula I as claimed in claim 3 wherein $R^1$ is 5-chloro, 6-chloro, 8-chloro, 6-bromo, 6-nitro or 7-nitro, n is 1 and $R^2$ is 3'-chloro or 3'-methyl, except that 5-chloro-4-(3'-chloroanilino)-, 6-chloro-4-(3'-methylanilino)- and 8-chloro-4-(3'-chloroanilino)-quinazoline or the pharmaceutically-acceptable salts thereof are excluded;
or a pharmaceutically-acceptable salt thereof.

**5.** A quinazoline derivative of the formula I as claimed in claim 3 wherein $R^1$ is hydrogen, n is 1 and $R^2$ is 2'- or 3'-methoxy;
or $R^1$ is hydrogen or chloro, n is 2 and each $R^2$, which may be the same or different, is chloro, methyl or methoxy, except that 4-(2',4'-dichloroanilino)-, 4-(2',6'-dimethylanilino)- and 4-(3',4'-dimethylanilino)-quinazoline or the pharmaceutically-acceptable salts thereof are excluded;
or a pharmaceutically-acceptable salt thereof.

**6.** A quinazoline derivative of the formula I as claimed in claim 3 selected from:-
4-(3'-methoxyanilino)quinazoline and 7-chloro-4-(4'-chloro-2'-methylanilino)quinazoline; or a pharmaceutically-acceptable acid-addition salt thereof.

**7.** A quinazoline derivative of the formula I as claimed in claim 3 selected from:-

4-(3'-bromoanilino)quinazoline,
4-(3'-iodoanilino)quinazoline,
6-chloro-4-(3'-chloroanilino)quinazoline,
6-bromo-4-(3'-methylanilino)quinazoline and
4-(3'-nitroanilino)quinazoline;

or a pharmaceutically-acceptable acid-addition salt thereof.

**8.** The use of a quinazoline derivative of the formula I, or a pharmaceutically-acceptable salt thereof, as defined in claim 1 or 2, as claimed in any one of claims 3 to 7, or wherein $R^1$ is hydrogen, halogeno, trifluoromethyl or nitro, n is 1 or 2 and each $R^2$, which may be the same or different, is hydrogen, halogeno, trifluoromethyl, nitro, cyano, amino, (1-4C)alkyl, (1-4C)alkoxy, N-(1-4C)alkylamino, N,N-di-[(1-4C)alkyl]amino, (1-4C)alkylthio, (1-4C)alkylsulphinyl or (1-4C)alkylsulphonyl, in the manufacture of a medicament for use in the production of an anti-cancer effect in a warm-blooded animal such as man.

**9.** The use of a quinazoline derivative of the formula I, or a pharmaceutically-acceptable salt thereof, as defined in claim 2, as claimed in claim 5 or claim 6, or wherein $R^1$ is hydrogen, halogeno, trifluoromethyl or nitro, n is 1 or 2 and each $R^2$, which may be the same or different, is hydrogen, halogeno, (1-4C)alkyl or (1-4C)alkoxy, in the manufacture of a medicament for use in the production of an anti-cancer effect in a warm-blooded animal such as man.

**10.** A process for the preparation of a quinazoline derivative of the formula I, or a pharmaceutically-acceptable salt thereof, as defined in claim 3 which comprises:-
the reaction of a quinazoline of the formula III

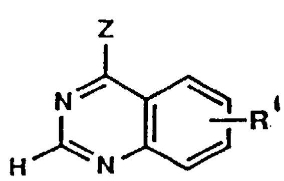

III

wherein Z is a displaceable group, with an aniline of the formula IV

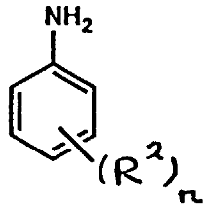

IV

and when a pharmaceutically-acceptable salt of a novel quinazoline derivative of the formula I is required, it may be obtained using a conventional procedure.

**Claims for the following Contracting State : GR**

**1.** A process for the preparation of a pharmaceutical composition characterised by bringing into association a quinazoline derivative of the formula I

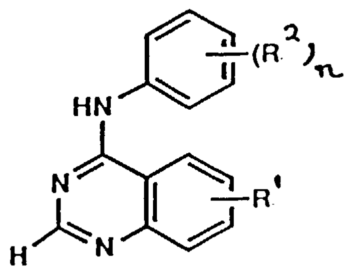

I

wherein $R^1$ is hydrogen, trifluoromethyl or nitro, n is 1 and $R^2$ is halogeno, trifluoromethyl, nitro, cyano, (1-4C) alkyl, (1-4C)alkoxy, N-(1-4C)alkylamino, N,N-di-[(1-4C)alkyl]amino, (1-4C)alkylthio, (1-4C)alkylsulphinyl or (1-4C)alkylsulphonyl, except that 7-trifluoromethyl-4-(4'-methoxyanilino)quinazoline or the pharmaceutically-acceptable salts thereof are excluded;
or wherein $R^1$ is 5-chloro, 6-chloro, 6-bromo or 8-chloro, n is 1 and $R^2$ is 3'-chloro or 3'-methyl, except that 5-chloro-4-(3'-chloroanilino)-, 6-chloro-4-(3'-methylanilino)- and 8-chloro-4-(3'-chloroanilino)-quinazoline or the pharmaceutically-acceptable salts thereof are excluded;
or wherein $R^1$ is hydrogen, halogeno, trifluoromethyl or nitro, n is 2 and each $R^2$, which may be the same or different, is halogeno, (1-4C)alkyl or (1-4C)alkoxy, except that 6-fluoro-4-(2',4'-dimethylanilino)quinazoline or the pharmaceutically-acceptable salts thereof are excluded;

or a pharmaceutically-acceptable salt thereof; together with a pharmaceutically-acceptable diluent or carrier.

**2.** A process for the preparation of a pharmaceutical composition characterised by bringing into association a quinazoline derivative of the formula I as defined in claim 1 wherein

$R^1$ is hydrogen, n is 1 and $R^2$ is halogeno, (1-4C)alkyl or (1-4C)alkoxy;
or $R^1$ is hydrogen, halogeno, trifluoromethyl or nitro, n is 2 and each $R^2$, which may be the same or different, is halogeno, (1-4C)alkyl or (1-4C)alkoxy, except that 6-fluoro-4-(2',4'-dimethylanilino) quinazoline or the pharmaceutically-acceptable salts thereof are excluded;

or a pharmaceutically-acceptable salt thereof; together with a pharmaceutically-acceptable diluent or carrier.

**3.** A process for the preparation of a quinazoline derivative of the formula I

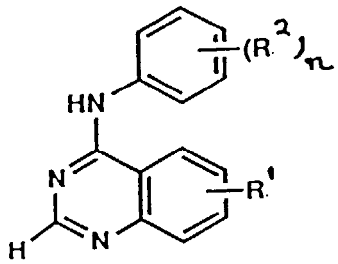

I

wherein $R^1$ is hydrogen, n is 1 and $R^2$ is 2'-methoxy, 3'-methoxy, 3'-fluoro, 3'-bromo, 3'-iodo, 3'-ethyl, 3'-nitro, 3'-cyano, 3'-methylthio or 3'-(N,N-dimethylamino);
or wherein $R^1$ is 5-chloro, 6-chloro, 8-chloro, 6-bromo or 7-nitro, n is 1 and $R^2$ is 3'-chloro or 3'-methyl, except that 5-chloro-4-(3'-chloroanilino)-, 6-chloro-4-(3'-methylanilino)- and 8-chloro-4-(3'-chloroanilino)-quinazoline or the pharmaceutically-acceptable salts thereof are excluded;
or wherein $R^1$ is hydrogen or chloro, n is 2 and each $R^2$, which may be the same or different, is chloro, methyl or methoxy, except that 4-(2',4'-dichloroanilino)-, 4-(2',6'-dimethylanilino)- and 4-(3',4'-dimethylanilino)-quinazoline or the pharmaceutically-acceptable salts thereof are excluded;

or a pharmaceutically-acceptable salt thereof;
characterised by
the reaction of a quinazoline of the formula III

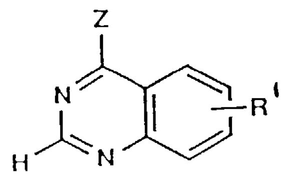

III

wherein Z is a displaceable group, with an aniline of the formula IV

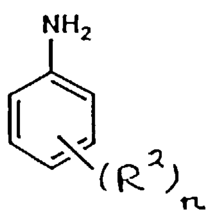

IV

and when a pharmaceutically-acceptable salt of a novel quinazoline derivative of the formula I is required, it may be obtained using a conventional procedure.

**4.** A process as claimed in claim 3 for the preparation of a quinazoline derivative of the formula I wherein $R^1$ is 5-chloro, 6-chloro, 8-chloro, 6-bromo, 6-nitro or 7-nitro, n is 1 and $R^2$ is 3'-chloro or 3'-methyl, except that 5-chloro-4-(3'-chloroanilino)-, 6-chloro-4-(3'-methylanilino)- and 8-chloro-4-(3'-chloroanilino)-quinazoline or the pharmaceutically-acceptable salts thereof are excluded;
or a pharmaceutically-acceptable salt thereof.

**5.** A process as claimed in claim 3 for the preparation of a quinazoline derivative of the formula I wherein $R^1$ is hydrogen, n is 1 and $R^2$ is 2'- or 3'-methoxy;
or $R^1$ is hydrogen or chloro, n is 2 and each $R^2$, which may be the same or different, is chloro, methyl or methoxy, except that 4-(2',4'-dichloroanilino)-, 4-(2',6'-dimethylanilino)- and 4-(3',4'-dimethylanilino)-quinazoline or the pharmaceutically-acceptable salts thereof are excluded;

or a pharmaceutically-acceptable salt thereof.

**6.** A process as claimed in claim 3 for the preparation of a quinazoline derivative of the formula I selected from:- 4-(3'-methoxyanilino)quinazoline and 7-chloro-4-(4'-chloro-2'-methylanilino)quinazoline; or a pharmaceutically-acceptable acid-addition salt thereof.

**7.** A process as claimed in claim 3 for the preparation of a quinazoline derivative of the formula I selected from:-

4-(3′-bromoanilino)quinazoline,
4-(3′-iodoanilino)quinazoline,
6-chloro-4-(3′-chloroanilino)quinazoline,
6-bromo-4-(3′-methylanilino)quinazoline and
4-(3′-nitroanilino)quinazoline;

or a pharmaceutically-acceptable acid-addition salt thereof.

**8.** The use of a quinazoline derivative of the formula I, or a pharmaceutically-acceptable salt thereof, as defined in in any one of claims 1 to 7, or wherein $R^1$ is hydrogen, halogeno, trifluoromethyl or nitro, n is 1 or 2 and each $R^2$, which may be the same or different, is hydrogen, halogeno, trifluoromethyl, nitro, cyano, amino, (1-4C)alkyl, (1-4C) alkoxy, N-(1-4C)alkylamino, N,N-di-[(1-4C)alkyl]amino, (1-4C)alkylthio, (1-4C)alkylsulphinyl or (1-4C)alkylsulphonyl, in the manufacture of a medicament for use in the production of an anti-cancer effect in a warm-blooded animal such as man.

**9.** The use of a quinazoline derivative of the formula I, or a pharmaceutically-acceptable salt thereof, as defined in claim 2, claim 5 or claim 6, or wherein $R^1$ is hydrogen, halogeno, trifluoromethyl or nitro, n is 1 or 2 and each $R^2$, which may be the same or different, is hydrogen, halogeno, (1-4C)alkyl or (1-4C)alkoxy, in the manufacture of a medicament for use in the production of an anti-cancer effect in a warm-blooded animal such as man.

**Patentansprüche**

**Patentansprüche für folgende Vertragsstaaten : AT, BE, CH, DE, DK, FR, GB, IT, LI, LU, MC, NL, PT, SE**

**1.** Pharmazeutische Zusammensetzung, die ein Chinazolin-Derivat mit der folgenden Formel I enthält:

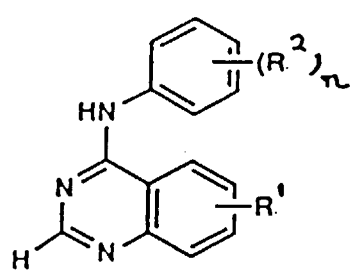

I

in der

$R^1$ für Wasserstoff, Trifluormethyl oder Nitro steht, n 1 ist und $R^2$ für Halogen, Trifluormethyl, Nitro, Cyano, (1-4C)Alkyl, (1-4C)Alkoxy, N-(1-4C)Alkylamino, N,N-Di-[(1-4C)alkyl]amino, (1-4C)Alkylthio, (1-4C)Alkylsulfinyl oder (1-4C)Alkylsulfonyl steht, wobei 7-Trifluormethyl-4-(4'-methoxyanilino)chinazolin oder die pharmazeutisch geeigneten Salze davon ausgenommen sind,

oder wobei $R^1$ für 5-Chlor, 6-Chlor, 6-Brom oder 8-Chlor steht, n 1 ist und $R^2$ für 3'-Chlor oder 3'-Methyl steht, wobei 5-Chlor-4-(3'-chloranilino)-, 6-Chlor-4-(3'-methylanilino)- und 8-Chlor-4-(3'-chloranilino)chinazolin oder die pharmazeutisch geeigneten Salze davon ausgenommen sind,

oder wobei $R^1$ für Wasserstoff, Halogen, Trifluormethyl oder Nitro steht, n 2 ist und jedes $R^2$, das gleich oder unterschiedlich sein kann, für Halogen, (1-4C)Alkyl oder (1-4C)Alkoxy steht, wobei 6-Fluor-4-(2',4'-dimethylanilino)chinazolin oder die pharmazeutisch geeigneten Salze davon ausgenommen sind,

oder ein pharmazeutisch geeignetes Salz davon, und einen pharmazeutisch geeigneten Streckstoff oder Trägerstoff.

**2.** Pharmazeutische Zusammensetzung, die ein Chinazolin-Derivat mit der in Anspruch 1 definierten Formel I enthält, wobei:

$R^1$ für Wasserstoff steht, n 1 ist und $R^2$ für Halogen, (1-4C)Alkyl oder (1-4C)Alkoxy steht, oder $R^1$ für Wasserstoff, Halogen, Trifluormethyl oder Nitro steht, n 2 ist und jedes $R^2$, das gleich oder unterschiedlich sein kann, für Halogen, (1-4C)Alkyl oder (1-4C)Alkoxy steht, wobei 6-Fluor-4-(2',4'-dimethylanilino)-chinazolin oder die pharmazeutisch geeigneten Salze davon ausgenommen sind,

oder ein pharmazeutisch geeignetes Salz davon, und einen pharmazeutisch geeigneten Streckstoff oder Trägerstoff.

**3.** Chinazolin-Derivat mit der Formel I:

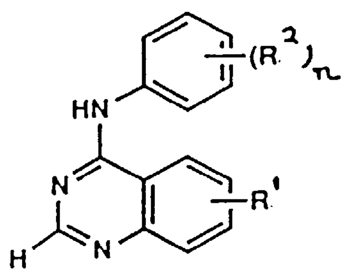

I

in der:
$R^1$ für Wasserstoff steht, n 1 ist und $R^2$ für 2'-Methoxy, 3'-Methoxy, 3'-Fluor, 3'-Brom, 3'-Jod, 3'-Ethyl, 3'-Nitro, 3'-Cyano, 3'-Methylthio oder 3'-(N,N-Dimethylamino) steht, oder wobei $R^1$ für 5-Chlor-, 6-Chlor, 8-Chlor, 6-Brom oder 7-Nitro steht, n 1 ist und $R^2$ für 3'-Chlor oder 3'-Methyl steht, wobei 5-Chlor-4-(3'-chloranilino)-, 6-Chlor-4-(3'-methylanilino)- und 8-Chlor-4-(3'-chloranilino)chinazolin oder deren pharmazeutisch geeigneten Salze ausgenommen sind, oder wobei $R^1$ für Wasserstoff oder Chlor steht, n 2 ist und jedes $R^2$, das gleich oder unterschiedlich sein kann, für Chlor, Methyl oder Methoxy steht, wobei 4-(2',4'-Dichloranilino)-, 4-(2',6'-Dimethylanilino)- und 4-(3',4'-Dimethylanilino)chinazolin oder deren pharmazeutisch geeigneten Salze davon ausgenommen sind, oder ein pharmazeutisch geeignetes Salz davon.

**4.** Chinazolin-Derivat mit der Formel I nach Anspruch 3, wobei
$R^1$ für 5-Chlor, 6-Chlor, 8-Chlor, 6-Brom, 6-Nitro oder 7-Nitro steht, n 1 ist und $R^2$ für 3'-Chlor oder 3'-Methyl steht, wobei 5-Chlor-4-(3'-chloranilino)-, 6-Chlor-4-(3'-methylanilino)- und 8-Chlor-4-(3'-chloranilino)chinazolin oder die pharmazeutisch geeigneten Salze davon ausgenommen sind, oder ein pharmazeutisch geeignetes Salz davon.

**5.** Chinazolin-Derivat mit der Formel I nach Anspruch 3, wobei
$R^1$ für Wasserstoff steht, n 1 ist und $R^2$ für 2'- oder 3'-Methoxy steht, oder $R^1$ für Wasserstoff oder Chlor steht, n 2 ist und jedes $R^2$, das gleich oder unterschiedlich sein kann, für Chlor, Methyl oder Methoxy steht, wobei 4-(2',4'-Dichloranilino)-, 4-(2',6'-Dimethylanilino)- und 4-(3',4'-Dimethylanilino)-chinazolin oder die pharmazeutisch geeigneten Salze davon ausgenommen sind, oder ein pharmazeutisch geeignetes Salz davon.

**6.** Chinazolin-Derivat mit der Formel I nach Anspruch 3, das ausgewählt ist unter:
4-(3'-Methoxyanilino)chinazolin und 7-Chlor-4-(4'-chlor-2'-methylanilino)chinazolin, oder einem pharmazeutisch geeigneten Säureadditionssalz davon.

**7.** Chinazolin-Derivat mit der Formel I nach Anspruch 3, das ausgewählt ist unter:

4-(3'-Bromanilino)chinazolin,
4-(3'-Jodanilino)chinazolin,
6-Chlor-4-(3'-chloranilino)chinazolin,
6-Brom-4-(3'-methylanilino)chinazolin und
4-(3'-Nitroanilino)chinazolin
oder einem pharmazeutisch geeigneten Säureadditionssalz davon.

**8.** Verwendung eines Chinazolin-Derivats mit der Formel I, oder eines pharmazeutisch geeigneten Salzes davon, wie in Anspruch 1 oder 2 definiert, nach einem der Ansprüche 3 bis 7, oder wobei
$R^1$ für Wasserstoff, Halogen, Trifluormethyl oder Nitro steht, n 1 oder 2 ist und jedes $R^2$, das gleich oder unterschiedlich sein kann, für Wasserstoff, Halogen, Trifluormethyl, Nitro, Cyano, Amino, (1-4C)Alkyl, (1-4C)Alkoxy, N-(1-4C)Alkylamino, N,N-Di-[(1-4C)-alkyl]amino, (1-4C)Alkylthio, (1-4C)Alkylsulfinyl oder (1-4C)Alkylsulfonyl steht, zur Herstellung eines Arzneimittels zur Erzeugung einer Antikrebswirkung an einem Warmblüter wie dem Menschen.

**9.** Verwendung eines Chinazolin-Derivats mit der Formel I, oder eines pharmazeutisch geeigneten Salzes davon, wie in Anspruch 2 definiert, nach Anspruch 5 oder 6, oder wobei
$R^1$ für Wasserstoff, Halogen, Trifluormethyl oder Nitro steht, n 1 oder 2 ist und jedes $R^2$, das gleich oder unterschiedlich sein kann, für Wasserstoff, Halogen, (1-4C)Alkyl oder (1-4C)Alkoxy steht, zur Herstellung eines Arzneimittels zur Erzeugung einer Antikrebswirkung in einem Warmblüter wie dem Menschen.

**10.** Verfahren zur Herstellung eines Chinazolin-Derivats mit der Formel I, oder eines pharmazeutisch geeigneten Salzes davon, wie in Anspruch 3 definiert, bei dem:
ein Chinazolin mit der Formel III

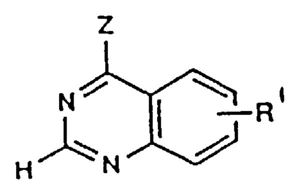

III

in der Z für eine substituierbare Gruppe steht, mit einem Anilin mit der Formel IV

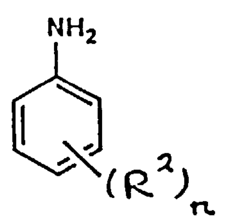

IV

umgesetzt wird, woraufhin, wenn ein pharmazeutisch geeignetes Salz eines neuen Chinazolin-Derivats mit der Formel I erforderlich ist, dieses unter Anwendung eines herkömmlichen Verfahrens erhältlich ist.

**Patentansprüche für folgenden Vertragsstaat : GR**

**1.** Verfahren zur Herstellung einer pharmazeutischen Zusammensetzung,
das dadurch **gekennzeichnet** ist,
daß folgendes miteinander in Verbindung gebracht wird:
ein Chinazolin-Derivat mit der folgenden Formel I:

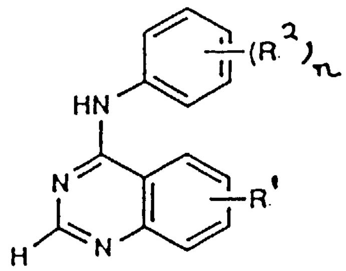

I

in der

$R^1$ für Wasserstoff, Trifluormethyl oder Nitro steht, n 1 ist und $R^2$ für Halogen, Trifluormethyl, Nitro, Cyano, (1-4C)Alkyl, (1-4C)Alkoxy, N-(1-4C)Alkylamino, N,N-Di-[(1-4C)alkyl]amino, (1-4C)Alkylthio, (1-4C)-Alkylsulfinyl oder (1-4C)Alkylsulfonyl steht, wobei 7-Trifluormethyl-4-(4'-methoxyanilino)chinazolin oder die pharmazeutisch geeigneten Salze davon ausgenommen sind,

oder wobei $R^1$ für 5-Chlor, 6-Chlor, 6-Brom oder 8-Chlor steht, n 1 ist und $R^2$ für 3'-Chlor oder 3'-Methyl steht, wobei 5-Chlor-4-(3'-chloranilino)-, 6-Chlor-4-(3'-methylanilino)- und 8-Chlor-4-(3'-chloranilino)chinazolin oder die pharmazeutisch geeigneten Salze davon ausgenommen sind,

oder wobei $R^1$ für Wasserstoff, Halogen, Trifluormethyl oder Nitro steht, n 2 ist und jedes $R^2$, das gleich oder unterschiedlich sein kann, für Halogen, (1-4C)Alkyl oder (1-4C)Alkoxy steht, wobei 6-Fluor-4-(2',4'-dimethylanilino)chinazolin oder die pharmazeutisch geeigneten Salze davon ausgenommen sind,

oder ein pharmazeutisch geeignetes Salz davon, und ein pharmazeutisch geeigneter Streckstoff oder Trägerstoff.

2. Verfahren zur Herstellung einer pharmazeutischen Zusammensetzung, das dadurch **gekennzeichnet** ist, daß folgendes miteinander in Verbindung gebracht wird:

ein Chinazolin-Derivat mit der in Anspruch 1 definierten Formel I, wobei

$R^1$ für Wasserstoff steht, n 1 ist und $R^2$ für Halogen, (1-4C)Alkyl oder (1-4C)Alkoxy steht, oder $R^1$ für Wasserstoff, Halogen, Trifluormethyl oder Nitro steht, n 2 ist und jedes $R^2$, das gleich oder unterschiedlich sein kann, für Halogen, (1-4C)Alkyl oder (1-4C)Alkoxy steht, wobei 6-Fluor-4-(2',4'-dimethylanilino)-chinazolin oder die pharmazeutisch geeigneten Salze davon ausgenommen sind,

oder ein pharmazeutisch geeignetes Salz davon, und ein pharmazeutisch geeigneter Streckstoff oder Trägerstoff.

3. Verfahren zur Herstellung eines Chinazolin-Derivats mit der Formel I:

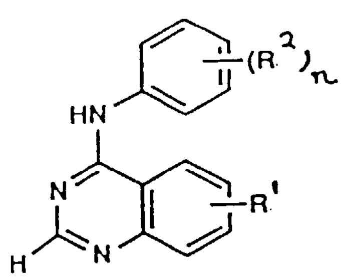

I

in der:

$R^1$ für Wasserstoff steht, n 1 ist und $R^2$ für 2'-Methoxy, 3'-Methoxy, 3'-Fluor, 3'-Brom, 3'-Jod, 3'-Ethyl, 3'-Nitro, 3'-Cyano, 3'-Methylthio oder 3'-(N,N-Dimethylamino) steht, oder wobei $R^1$ für 5-Chlor-, 6-Chlor, 8-Chlor, 6-Brom oder 7-Nitro steht, n 1 ist und $R^2$ für 3'-Chlor oder 3'-Methyl steht, wobei 5-Chlor-4-(3'-chloranilino)-, 6-Chlor-4-(3'-methylanilino)- und 8-Chlor-4-(3'-chloranilino)chinazolin oder deren pharmazeutisch geeigneten Salze ausgenommen sind, oder wobei $R^1$ für Wasserstoff oder Chlor steht, n 2 ist und jedes $R^2$, das gleich oder unterschiedlich sein kann, für Chlor, Methyl oder Methoxy steht, wobei 4-(2',4'-Dichloranilino)-, 4-(2',6'-Dimethylanilino)- und 4-(3',4'-Dimethylanilino)chinazolin oder deren pharmazeutisch geeigneten Salze davon ausgenommen sind, oder eines pharmazeutisch geeigneten Salzes davon,
dadurch **gekennzeichnet**, daß
ein Chinazolin mit der Formel III

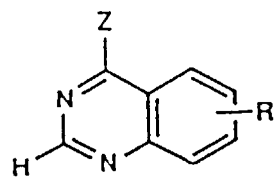

III

in der Z für eine substituierbare Gruppe steht, mit einem Anilin mit der Formel IV

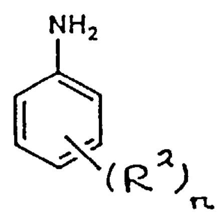

IV

umgesetzt wird, woraufhin, wenn ein pharmazeutisch geeignetes Salz eines neuen Chinazolin-Derivats mit der Formel I erforderlich ist, dieses unter Anwendung eines herkömmlichen Verfahrens erhältlich ist.

**4.** Verfahren nach Anspruch 3 zur Herstellung eines Chinazolin-Derivats mit der Formel I,
wobei
$R^1$ für 5-Chlor, 6-Chlor, 8-Chlor, 6-Brom, 6-Nitro oder 7-Nitro steht, n 1 ist und $R^2$ für 3'-Chlor oder 3'-Methyl steht, wobei 5-Chlor-4-(3'-chloranilino)-, 6-Chlor-4-(3'-methylanilino)- und 8-Chlor-4-(3'-chlor-anilino)chinazolin oder die pharmazeutisch geeigneten Salze davon ausgenommen sind, oder eines pharmazeutisch geeigneten Salzes davon.

**5.** Verfahren nach Anspruch 3 zur Herstellung eines Chinazolin-Derivats mit der Formel I,
wobei
$R^1$ für Wasserstoff steht, n 1 ist und $R^2$ für 2'- oder 3'-Methoxy steht, oder $R^1$ für Wasserstoff oder Chlor steht, n 2 ist und jedes $R^2$, das gleich oder unterschiedlich sein kann, für Chlor, Methyl oder Methoxy steht, wobei 4-(2',4'-Dichloranilino)-, 4-(2',6'-Dimethylanilino)- und 4-(3',4'-Dimethylanilino)-chinazolin oder die pharmazeutisch geeigneten Salze davon ausgenommen sind, oder eines pharmazeutisch geeigneten Salzes davon.

**6.** Verfahren nach Anspruch 3 zur Herstellung eines Chinazolin-Derivats mit der Formel I, das unter folgenden ausgewählt ist:
4-(3'-Methoxyanilino)chinazolin und 7-Chlor-4-(4'-chlor-2'-methylanilino)chinazolin, oder einem pharmazeutisch geeigneten Säureadditionssalz davon.

**7.** Verfahren nach Anspruch 3 zur Herstellung eines Chinazolin-Derivats mit der Formel I, das ausgewählt ist unter:

4-(3'-Bromanilino)chinazolin,

4-(3'-Jodanilino)chinazolin,
6-Chlor-4-(3'-chloranilino)chinazolin,
6-Brom-4-(3'-methylanilino)chinazolin und
4-(3'-Nitroanilino)chinazolin
oder einem pharmazeutisch geeigneten Säureadditionssalz davon.

**8.** Verwendung eines Chinazolin-Derivats mit der Formel I, oder eines pharmazeutisch geeigneten Salzes davon, wie in einem der Ansprüche 1 bis 7 definiert, oder wobei
$R^1$ für Wasserstoff, Halogen, Trifluormethyl oder Nitro steht, n 1 oder 2 ist und jedes $R^2$, das gleich oder unterschiedlich sein kann, für Wasserstoff, Halogen, Trifluormethyl, Nitro, Cyano, Amino, (1-4C)Alkyl, (1-4C)Alkoxy, N-(1-4C)Alkylamino, N,N-Di-[(1-4C)alkyl]amino, (1-4C)Alkylthio, (1-4C)Alkyl-sulfinyl oder (1-4C)Alkylsulfonyl steht, zur Herstellung eines Arzneimittels zur Erzeugung einer Antikrebswirkung an einem Warmblüter wie dem Menschen.

**9.** Verwendung eines Chinazolin-Derivats mit der Formel I, oder eines pharmazeutisch geeigneten Salzes davon, wie in Anspruch 2, 5 oder 6 definiert, oder wobei
$R^1$ für Wasserstoff, Halogen, Trifluormethyl oder Nitro steht, n 1 oder 2 ist und jedes $R^2$, das gleich oder unterschiedlich sein kann, für Wasserstoff, Halogen, (1-4C)Alkyl oder (1-4C)Alkoxy steht, zur Herstellung eines Arzneimittels zur Erzeugung einer Antikrebswirkung in einem Warmblüter wie dem Menschen.

**Revendications**

**Revendications pour les Etats contractants suivants : AT, BE, CH, DE, DK, FR, GB, IT, LI, LU, MC, NL, PT, SE**

**1.** Composition pharmaceutique qui comprend un dérivé de quinazoline de formule I

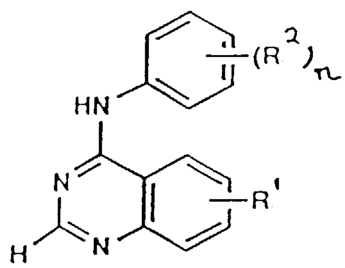

I

dans laquelle $R^1$ représente l'hydrogène, un groupe trifluorométhyle ou nitro, n est égal à 1 et $R^2$ représente un groupe halogéno, trifluorométhyle, nitro, cyano, alkyle en $C_1$ à $C_4$, alkoxy en $C_1$ à $C_4$, N-(alkyle en $C_1$ à $C_4$)amino, N,N-di(alkyle en $C_1$ à $C_4$)amino, alkylthio en $C_1$ à $C_4$, alkysulfinyle en $C_1$ à $C_4$ ou alkylsulfonyle en $C_1$ à $C_4$, à l'exclusion de la 7-trifluorométhyl-4-(4'-méthoxyanilino)quinazoline ou de ses sels pharmaceutiquement acceptables ;

ou dans laquelle $R^1$ représente un groupe 5-chloro, 6-chloro, 6-bromo ou 8-chloro, n est égal à 1 et $R^2$ représente un groupe 3'-chloro ou 3'-méthyle, à l'exclusion des 5-chloro-4-(3'-chloranilino)-, 6-chloro-4-(3'-méthylanilino)- et 8-chloro-4-(3'-chloranilino)-quinazolines ou de leurs sels pharmaceutiquement acceptables ;
ou dans laquelle $R^1$ représente l'hydrogène, un groupe halogéno, trifluorométhyle ou nitro, n est égal à 2 et les groupes $R^2$, qui peuvent être identiques ou différents, représentent chacun un groupe halogéno, alkyle en $C_1$ à $C_4$, alkoxy en $C_1$ à $C_4$, à l'exclusion de la 6-fluoro-4-(2',4'-diméthylanilino)quinazoline ou de ses sels pharmaceutiquement acceptables ;
ou un de sels pharmaceutiquement acceptables ; en association avec un diluant ou support pharmaceutiquement acceptable.

**2.** Composition pharmaceutique qui comprend un dérivé de quinazoline de formule I répondant à la définition suivant la revendication 1, dans laquelle $R^1$ représente l'hydrogène, n est égal à 1 et $R^2$ représente un groupe halogéno, alkyle en $C_1$ à $C_4$ ou alkoxy en $C_1$ à $C_4$ ;

ou $R^1$ représente l'hydrogène, un groupe halogéno, trifluorométhyle ou nitro, n est égal à 2 et les groupes $R^2$, qui peuvent être identiques ou différents, représentent chacun un groupe halogéno, alkyle en $C_1$ à $C_4$ ou alkoxy en $C_1$ à $C_4$, à l'exclusion de la 6-fluoro-4-(2',4'-diméthylanilino)-quinazoline ou de ses sels pharmaceutiquement acceptables ;

ou un de ses sels pharmaceutiquement acceptables ; en association avec un diluant ou support pharmaceutiquement acceptable.

3. Dérivé de quinazoline de formule I

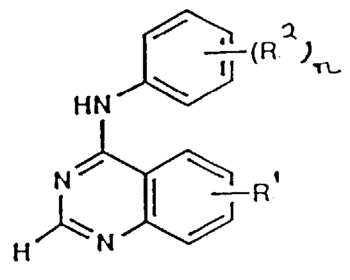

I

dans laquelle $R^1$ représente l'hydrogène, n est égal à 1 et $R^2$ représente un groupe 2'-méthoxy, 3'-méthoxy, 3'-fluoro, 3'-bromo, 3'-iodo, 3'-éthyle, 3'-nitro, 3'-cyano, 3'-méthylthio ou 3'-(N,N-diméthylamino) ;

ou dans laquelle $R^1$ représente un groupe 5-chloro, 6-chloro, 8-chloro, 6-bromo ou 7-nitro, n est égal à 1 et $R^2$ représente un groupe 3'-chloro ou 3'-méthyle, à l'exclusion des 5-chloro-4-(3'-chloranilino)-, 6-chloro-4-(3'-méthylanilino)- et 8-chloro-4-(3'-chloranilino) -quinazolines ou de leurs sels pharmaceutiquement acceptables ;

ou dans laquelle $R^1$ représente l'hydrogène ou un groupe chloro, n est égal à 2 et les groupes $R^2$, qui peuvent être identiques ou différents, représentent chacun un groupe chloro, méthyle ou méthoxy, à l'exclusion des 4-(2',4'-dichloranilino)-, 4-(2',6'-diméthylanilino)- et 4-(3',4'-diméthylanilino)-quinazolines ou de leurs sels pharmaceutiquement acceptables ;

ou un de ses sels pharmaceutiquement acceptables.

4. Dérivé de quinazoline de formule I suivant la revendication 3, dans laquelle $R^1$ représente un groupe 5-chloro, 6-chloro, 8-chloro, 6-bromo, 6-nitro ou 7-nitro, n est égal à 1 et $R^2$ représente un groupe 3'-chloro ou 3'-méthyle, à l'exclusion des 5-chloro-4-(3'-chloranilino)-, 6-chloro-4-(3'-méthylanilino)- et 8-chloro-4-(3'-chloranilino)-quinazolines ou de leurs sels pharmaceutiquement acceptables ;

ou un de ses sels pharmaceutiquement acceptables.

5. Dérivé de quinazoline de formule I suivant la revendication 3, dans laquelle $R^1$ représente l'hydrogène, n est égal à 1 et $R^2$ représente un groupe 2'- ou 3'-méthoxy ;

ou $R^1$ représente l'hydrogène ou un groupe chloro, n est égal à 2 et les groupes $R^2$, qui peuvent être identiques ou différents, représentent chacun un groupe chloro, méthyle ou méthoxy, à l'exclusion des 4-(2',4'-dichloranilino)-, 4-(2',6'-diméthylanilino)- et 4-(3',4'-diméthylanilino)-quinazolines ou de leurs sels pharmaceutiquement acceptables ;

ou un de ses sels pharmaceutiquement acceptables.

6. Dérivé de quinazoline de formule I suivant la revendication 3, choisi entre :

la 4-(3'-méthoxyanilino)quinazoline et la 7-chloro-4-(4'-chloro-2'-méthylanilino)quinazoline ; ou un de ses sels d'addition d'acides pharmaceutiquement acceptables.

7. Dérivé de quinazoline de formule I suivant la revendication 3, choisi entre :

la 4-(3'-bromoanilino)quinazoline,
la 4-(3'-iodoanilino)quinazoline,
la 6-chloro-4-(3'-chloranilino)quinazoline,
la 6-bromo-4-(3'-méthylanilino)quinazoline et

la 4-(3'-nitroanilino)quinazoline ;
ou un de ses sels d'addition d'acides pharmaceutiquement acceptables.

**8.** Utilisation d'un dérivé de quinazoline de formule I, ou d'un de ses sels pharmaceutiquement acceptables, répondant à la définition suivant la revendication 1 ou 2, revendiqué dans l'une quelconque des revendications 3 à 7, ou dans lequel $R^1$ représente l'hydrogène, un groupe halogéno, trifluorométhyle ou nitro, n est égal à 1 ou 2 et les groupes $R^2$, qui peuvent être identiques ou différents, représentent chacun l'hydrogène, un groupe halogéno, trifluorométhyle, nitro, cyano, amino, alkyle en $C_1$ à $C_4$, alkoxy en $C_1$ à $C_4$, N-(alkyle en $C_1$ à $C_4$)amino, N,N-di(alkyle en $C_1$ à $C_4$)amino, alkylthio en $C_1$ à $C_4$, alkysulfinyle en $C_1$ à $C_4$ ou alkylsulfonyle en $C_1$ à $C_4$, dans la production d'un médicament destiné à être utilisé pour engendrer un effet anticancéreux chez un animal à sang chaud tel que l'homme.

**9.** Utilisation d'un dérivé de quinazoline de formule I, ou d'un des ses sels pharmaceutiquement acceptables, répondant à la définition suivant la revendication 2, revendiqué dans la revendication 5 ou la revendication 6, ou dans lequel $R^1$ représente l'hydrogène, un groupe halogéno, trifluorométhyle ou nitro, n est égal à 1 ou 2 et les groupes $R^2$, qui peuvent être identiques ou différents, représentent chacun l'hydrogène, un groupe halogéno, alkyle en $C_1$ à $C_4$ ou alkoxy en $C_1$ à $C_4$, dans la production d'un médicament destiné à être utilisé pour engendrer un effet anticancéreux chez un animal à sang chaud tel que l'homme.

**10.** Procédé pour la préparation d'un dérivé de quinazoline de formule I, ou d'un de ses sels pharmaceutiquement acceptables, répondant à la définition suivant la revendication 3, qui comprend :
la réaction d'une quinazoline de formule III

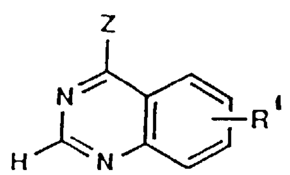

III

dans laquelle Z représente un groupe déplaçable, avec une aniline de formule IV

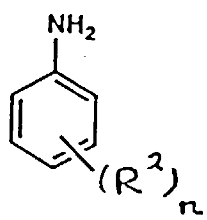

IV

et, lorsqu'un sel pharmaceutiquement acceptable d'un dérivé de quinazoline nouveau de formule I est requis, la possibilité d'obtention de ce sel par un mode opératoire classique.

**Revendications pour l'Etat contractant suivant : GR**

**1.** Procédé de préparation d'une composition pharmaceutique, caractérisé par la mise en association d'un dérivé de quinazoline de formule I

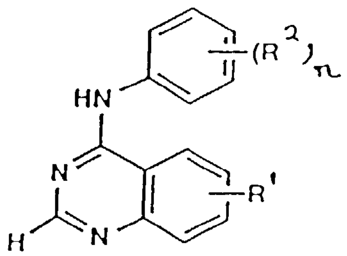

I

dans laquelle $R^1$ représente l'hydrogène, un groupe trifluorométhyle ou nitro, n est égal à 1 et $R^2$ représente un groupe halogéno, trifluorométhyle, nitro, cyano, alkyle en $C_1$ à $C_4$, alkoxy en $C_1$ à $C_4$, N-(alkyle en $C_1$ à $C_4$)amino, N,N-di(alkyle en $C_1$ à $C_4$)amino, alkylthio en $C_1$ à $C_4$, alkysulfinyle en $C_1$ à $C_4$ ou alkylsulfonyle en $C_1$ à $C_4$, à l'exclusion de la 7-trifluorométhyl-4-(4'-méthoxyanilino)quinazoline ou de ses sels pharmaceutiquement acceptables ;

ou dans laquelle $R^1$ représente un groupe 5-chloro, 6-chloro, 6-bromo ou 8-chloro, n est égal à 1 et $R^2$ représente un groupe 3'-chloro ou 3'-méthyle, à l'exclusion des 5-chloro-4-(3'-chloranilino)-, 6-chloro-4-(3'-méthylanilino)- et 8-chloro-4-(3'-chloranilino)-quinazolines ou de leurs sels pharmaceutiquement acceptables ;
ou dans laquelle $R^1$ représente l'hydrogène, un groupe halogéno, trifluorométhyle ou nitro, n est égal à 2 et les groupes $R^2$, qui peuvent être identiques ou différents, représentent chacun un groupe halogéno, alkyle en $C_1$ à $C_4$, alkoxy en $C_1$ à $C_4$, à l'exclusion de la 6-fluoro-4-(2',4'-diméthylanilino)quinazoline ou de ses sels pharmaceutiquement acceptables ;
ou d'un de ses sels pharmaceutiquement acceptables ; avec un diluant ou support pharmaceutiquement acceptable.

**2.** Procédé pour la préparation d'une composition pharmaceutique, caractérisé par la mise en association d'un dérivé de quinazoline de formule I répondant à la définition suivant la revendication 1, dans laquelle $R^1$ représente l'hydrogène, n est égal à 1 et $R^2$ représente un groupe halogéno, alkyle en $C_1$ à $C_4$ ou alkoxy en $C_1$ à $C_4$ ;

ou $R^1$ représente l'hydrogène, un groupe halogéno, trifluorométhyle ou nitro, n est égal à 2 et les groupes $R^2$, qui peuvent être identiques ou différents, représentent chacun un groupe halogéno, alkyle en $C_1$ à $C_4$ ou alkoxy en $C_1$ à $C_4$, à l'exclusion de la 6-fluoro-4-(2',4'-diméthylanilino)-quinazoline ou de ses sels pharmaceutiquement acceptables ;
ou d'un de ses sels pharmaceutiquement acceptables ; avec un diluant ou support pharmaceutiquement acceptable.

**3.** Procédé pour la préparation d'un dérivé de quinazoline de formule I

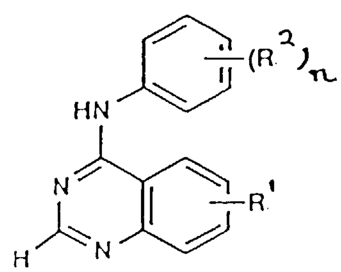

I

dans laquelle $R^1$ représente l'hydrogène, n est égal à 1 et $R^2$ représente un groupe 2'-méthoxy, 3'-méthoxy, 3'-fluoro, 3'-bromo, 3'-iodo, 3'-éthyle, 3'-nitro, 3'-cyano, 3'-méthylthio ou 3'-(N,N-diméthylamino) ;

ou dans laquelle $R^1$ représente un groupe 5-chloro, 6-chloro, 8-chloro, 6-bromo ou 7-nitro, n est égal à 1 et $R^2$ représente un groupe 3'-chloro ou 3'-méthyle, à l'exclusion des 5-chloro-4-(3'-chloranilino) -, 6-chloro-4-(3'-méthylanilino)- et 8-chloro-4-(3'-chloranilino)-quinazolines ou de leurs sels pharmaceutiquement acceptables ;

ou dans laquelle $R^1$ représente l'hydrogène ou un groupe chloro, n est égal à 2 et les groupes $R^2$, qui peuvent être identiques ou différents, représentent chacun un groupe chloro, méthyle ou méthoxy, à l'exclusion des 4-(2',4'-dichloranilino)-, 4-(2',6'-diméthylanilino)- et 4-(3',4'-diméthylanilino)-quinazolines ou de leurs sels pharmaceutiquement acceptables ;
ou d'un de ses sels pharmaceutiquement acceptables ;

caractérisé par
la réaction d'une quinazoline de formule III

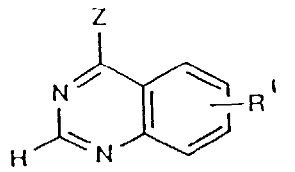

III

dans laquelle Z représente un groupe déplaçable, avec une aniline de formule IV

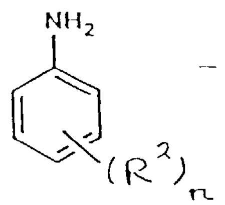

IV

et, lorsqu'un sel pharmaceutiquement acceptable d'un dérivé de quinazoline nouveau de formule I est requis, la possibilité d'obtention de ce sel par un mode opératoire classique.

**4.** Procédé suivant la revendication 3 pour la préparation d'un dérivé de quinazoline de formule I dans laquelle $R^1$ représente un groupe 5-chloro, 6-chloro, 8-chloro, 6-bromo, 6-nitro ou 7-nitro, n est égal à 1 et $R^2$ représente un groupe 3'-chloro ou 3'-méthyle, à l'exclusion des 5-chloro-4-(3'-chloranilino)-, 6-chloro-4-(3'-méthyl-anilino)- et 8-chloro-4-(3'-chloranilino)-quinazolines ou de leurs sels pharmaceutiquement acceptables ;
ou d'un de ses sels pharmaceutiquement acceptables.

**5.** Procédé suivant la revendication 3 pour la préparation d'un dérivé de quinazoline de formule I dans laquelle $R^1$ représente l'hydrogène, n est égal à 1 et $R^2$ représente un groupe 2'- ou 3'-méthoxy ;

ou $R^1$ représente l'hydrogène ou un groupe chloro, n est égal à 2 et les groupes $R^2$, qui peuvent être identiques ou différents, représentent chacun un groupe chloro, méthyle ou méthoxy, à l'exclusion des 4-(2',4'-dichloranilino)-, 4-(2',6'-diméthylanilino)- et 4-(3',4'-diméthylanilino)-quinazolines ou de leurs sels pharmaceutiquement acceptables ;
ou d'un de ses sels pharmaceutiquement acceptables.

**6.** Procédé suivant la revendication 3 pour la préparation d'un dérivé de quinazoline de formule I choisi entre :
la 4-(3'-méthoxyanilino)quinazoline et la 7-chloro-4-(4'-chloro-2'-méthylanilino)quinazoline ; ou d'un de ses sels d'addition d'acides pharmaceutiquement acceptables.

**7.** Procédé suivant la revendication 3 pour la préparation d'un dérivé de quinazoline de formule I choisi entre :

la 4-(3'-bromoanilino)quinazoline,
la 4-(3'-iodoanilino)quinazoline,
la 6-chloro-4-(3'-chloranilino)quinazoline,
la 6-bromo-4-(3'-méthylanilino)quinazoline et
la 4-(3'-nitroanilino)quinazoline ;

ou d'un de ses sels d'addition d'acides pharmaceutiquement acceptables.

**8.** Utilisation d'un dérivé de quinazoline de formule I, ou d'un de ses sels pharmaceutiquement acceptables, répondant à la définition suivant l'une quelconque des revendications 1 à 7, ou dans lequel $R^1$ représente l'hydrogène, un groupe halogéno, trifluorométhyle ou nitro, n est égal à 1 ou 2 et les groupes $R^2$, qui peuvent être identiques ou différents, représentent chacun l'hydrogène, un groupe halogéno, trifluorométhyle, nitro, cyano, amino, alkyle en $C_1$ à $C_4$, alkoxy en $C_1$ à $C_4$, N-(alkyle en $C_1$ à $C_4$)amino, N,N-di(alkyle en $C_1$ à $C_4$)amino, alkylthio en $C_1$ à $C_4$, alkysulfinyle en $C_1$ à $C_4$ ou alkylsulfonyle en $C_1$ à $C_4$, dans la production d'un médicament destiné à être utilisé pour engendrer un effet anticancéreux chez un animal à sang chaud tel que l'homme.

**9.** Utilisation d'un dérivé de quinazoline de formule I, ou d'un des ses sels pharmaceutiquement acceptables, répondant à la définition suivant la revendication 2, la revendication 5 ou la revendication 6, ou dans lequel $R^1$ représente l'hydrogène, un groupe halogéno, trifluorométhyle ou nitro, n est égal à 1 ou 2 et les groupes $R^2$, qui peuvent être identiques ou différents, représentent chacun l'hydrogène, un groupe halogéno, alkyle en $C_1$ à $C_4$ ou alkoxy en $C_1$ à $C_4$, dans la production d'un médicament destiné à être utilisé pour engendrer un effet anticancéreux chez un animal à sang chaud tel que l'homme.